# FASCICULE DE BREVET EUROPEEN

(11) **EP 2 821 052 B1**
(45) Date de publication et mention de la délivrance du brevet: **11.01.2017**
(21) Numéro de dépôt: 14166076.1
(22) Date de dépôt: 25.04.2014
(51) Int. Cl.: A61K 8/33, A61Q 5/00, A61K 8/55, A61Q 5/02, A61Q 5/12, A61Q 7/00, A61K 8/68, A61K 8/97, A61K 8/14

(54) **Association de miliacine et de lipides polaires, notamment de sphingolipides et/ou de phospholipides, pour le soin des cheveux et du cuir chevelu**
Verbindung von Miliacin und polaren Lipiden, insbesondere Sphingolipiden und/oder Phospholipiden, zur Pflege der Haare und der Kopfhaut
Combination of miliacin and polar lipids, in particular of sphingolipids and/or phospholipids, for care of the hair and scalp

(30) Priorité: 26.04.2013 FR 1353864
(43) Date de publication de la demande: 07.01.2015
(73) Titulaire: Etablissements Arco, 06130 Grasse (FR)
(72) Inventeur: Lamay, François, 56000 VANNES (FR); Lamour, Irène, 56000 VANNES (FR); Gaillard, Emmanuelle, 56690 LANDEVANT (FR)
(74) Mandataire: Novagraaf Technologies

(56) Documents cités:
- WO-A1-95/05146
- DE-A1- 4 113 346
- DE-A1- 19 508 628
- DE-A1-102005 017 147

## Description

### 1. Le domaine de l'invention

La présente invention concerne le domaine du soin cosmétique des matières kératiniques, notamment les cheveux et le cuir chevelu humains. L'invention trouve également son application pour le traitement cosmétique des poils d'animaux, notamment les animaux de compagnie et les animaux domestiques.

En particulier, l'invention concerne une composition pour administration orale ou pour application cutanée, comprenant une association d'actifs destinée à lutter contre la chute des cheveux et/ou favoriser leur croissance et/ou leur repousse et/ou améliorer leur beauté (brillance, douceur, force) et/ou améliorer le confort du cuir chevelu ou, chez l'animal, lutter contre une chute excessive de poils, et/ou favoriser leur croissance et ou améliorer leur beauté.

L'invention concerne encore l'utilisation d'une telle composition, ainsi que le procédé de préparation d'une telle composition.

### Le cheveu

Le cheveu est composé d'une tige qui est la partie visible, d'un bulbe pilaire situé à 3 ou 4 mm sous la surface du cuir chevelu et d'une papille dermique. La tige est composée à 95 % de kératine, d'eau, d'acides gras, de mélanine et d'une infime quantité de fer et de zinc.

La tige est entourée de deux gaines : la gaine épithéliale interne nécessaire au développement du cheveu, et la gaine épithéliale externe de composition voisine à celle de l'épiderme. Elles déterminent la forme définitive du cheveu. La gaine épithéliale externe héberge en particulier les cellules souches à partir desquelles le follicule pileux sera cycliquement régénéré.

Sur toute sa surface la fibre est protégée par une mince couche appelée cuticule. Elle est formée de cellules incolores nommées écailles qui se chevauchent. La cohésion entre ces écailles est assurée par un ciment riche en lipides et notamment en céramides.

Le bulbe est composé de kératinocytes qui se divisent activement pour former la tige pilaire ainsi que les gaines épithéliales interne et externe.

La papille dermique à la base du bulbe permet une irrigation, une oxygénation du cheveu et l'évacuation des déchets cellulaires. Elle est constituée de fibroblastes qui sécrètent une importante matrice extracellulaire. Très vascularisée, elle participe aux fonctions de nutrition et de régulation du cheveu. La papille peut être considérée comme le moteur biologique du cheveu.

### Cycle pilaire

Les cheveux naissent, croissent et chutent selon un cycle pilaire indépendant comprenant les phases suivantes.

La phase anagène est la phase de croissance du cheveu, pendant laquelle le cheveu croît à partir du bulbe. Les cellules de la matrice pilaire se divisent fortement et forment la tige pilaire et les gaines épithéliales internes et externes. En phase anagène, la matrice pilaire est le lieu d'une prolifération cellulaire intense. La tige du cheveu est ensuite kératinisée. Simultanément, le follicule pileux croît dans les couches profondes de la peau afin de nourrir le cheveu. Il s'agit de la période la plus longue du cycle pilaire puisqu'elle dure en moyenne 2 à 5 ans. La très grande majorité des cheveux est en phase anagène.

La phase catagène est une phase de repos pendant laquelle le cheveu cesse d'évoluer. Cette phase dure environ 3 semaines.

Enfin, pendant la phase télogène, le cheveu ne pousse plus, mais il reste attaché au follicule pileux. À la fin de cette phase, l'ancien cheveu tombe et laisse place à un nouveau follicule en phase anagène, le cycle pilaire recommence. Pendant cette phase, la prolifération cellulaire est faible ou nulle, on n'observe plus de croissance de la tige pilaire.

Lorsque le cycle pilaire est normal, le pourcentage de cheveux dans la phase anagène est compris entre 85 et 90% de l'ensemble des cheveux, la phase catagène concerne environ 1% des cheveux et environ 10 à 15% des cheveux sont en phase télogène.

Mais il peut arriver, au cours de la vie d'un homme ou d'une femme, pour des causes diverses, que le cycle pilaire soit modifié de façon anormale. Les causes de telles modifications peuvent être notamment le changement de saison, la fatigue, le stress, un déséquilibre hormonal, un traitement des cheveux, un traitement médicamenteux ayant un effet sur les cheveux, la pollution ou encore le vieillissement. Dans ce cas, le pourcentage de cheveux en phase anagène peut descendre en deçà de 80% de l'ensemble des cheveux et les cheveux en phase télogène peuvent être dans une proportion supérieure à 20% de l'ensemble des cheveux. La perte des cheveux devient excessive. Cette perte anormale de cheveux s'accompagne d'une fragilisation de la structure du cheveu.

De la même manière, certains animaux à poils, tels que des animaux de compagnie, subissent parfois, ponctuellement ou de manière prolongée, une chute anormalement importante de leurs poils.

### 2. Solutions de l'art antérieur

Pour remédier à tout ou partie de ce problème de cycle pilaire déséquilibré conduisant à une chute anormalement élevée de cheveux et à une fragilisation des cheveux, certains médicaments, produits cosmétiques ou compléments alimentaires sont connus.

En particulier, on connaît des principes actifs de médicaments tels que le minoxidil et la finastéride (ou propecia). Ces principes actifs ont une réelle efficacité mais présentent l'inconvénient d'être des produits chimiques, pouvant, comme toutes molécules de synthèse, produire des effets secondaires gênants chez le patient tels que des irritations ou l'épaississement des poils, entre autres. De plus, la finastéride est déconseillée aux femmes.

On connaît par ailleurs des compléments alimentaires à base de vitamines et de minéraux, pour lesquels un effet sur la beauté des phanères et de la peau est annoncé, sans particulièrement cibler un traitement « antichute » des cheveux. Cependant, ces vitamines et minéraux sont apportés facilement dans le cadre d'une alimentation variée et équilibrée.

D'autres compléments alimentaires contenant des extraits végétaux associés ou non à des vitamines et minéraux existent. Ils sont pour la plupart utilisés de façon traditionnelle, mais sans qu'il existe d'études cliniques prouvant leur efficacité. On peut citer entre autres l'huile de pépins de courge, la levure de bière, le thé vert, les isoflavones de soja.

D'autres compléments alimentaires ne concernent que la chute de cheveux excessive liée à un déséquilibre hormonal notamment l'alopécie androgénétique. C'est par exemple le cas des compléments alimentaires à base de Saw-palmetto. Ces compléments alimentaires sont principalement destinés aux hommes.

Un autre complément alimentaire connu, commercialisé sous la marque Priorin ® par la société Bayer, favorise la pousse du cheveu en fournissant aux cellules les nutriments essentiels. Il contient un extrait total de millet d'or et de l'huile de germe de blé, associés à de la cystine et à une vitamine indispensable au cheveu, le pantothénate de calcium. L'huile de germe de blé est composée d'acides gras tels que l'acide linoléique et de vitamine E.

Encore un autre complément alimentaire commercialisé sous la dénomination Hirsana ® par la société Zwicky AG est basé sur l'huile de millet contenant de la miliacine associée à des vitamines et minéraux pour contribuer à stopper la chute des cheveux, et rendre cheveux et ongles plus résistants.

### 3. Objectifs de l'invention

L'invention vise à fournir une composition originale, utilisée en cosmétique ou en complément alimentaire, qui soit réellement efficace et ciblée dans la lutte contre la chute des cheveux ou des poils d'animaux et/ou pour favoriser leur croissance et/ou leur repousse et/ou améliorer leur beauté (brillance, douceur, force) et /ou améliorer le confort du cuir chevelu.

L'invention vise de plus à fournir une telle composition qui n'intègre que des extraits végétaux naturels.

L'invention a encore pour objectif de fournir une telle composition qui ne provoque pas d'effets secondaires gênants et qui ne modifie pas l'équilibre hormonal de la personne ou de l'animal concerné.

### 4. Exposé de l'invention

L'invention répond à tout ou partie des objectifs précités grâce à une composition cosmétique ou alimentaire pour lutter contre la chute de cheveux ou de poils d'animaux et/ou pour favoriser leur croissance et/ou leur repousse et/ou améliorer leur beauté (brillance, douceur, force) et /ou améliorer le confort du cuir chevelu, comprenant au moins de la miliacine dans une proportion supérieure à 0,01 %, notamment 0,1 % massique par rapport à la masse totale de la composition et des lipides polaires dans une proportion supérieure à 0,1% massique par rapport à la masse totale de la composition, dont des sphingolipides, notamment des céramides et/ou des glycosylcéramides, et/ou des phospholipides dans une proportion supérieure à 0,1% massique par rapport à la masse totale de la composition.

Grâce à l'invention, on dispose d'une composition à base de produits naturels, particulièrement efficace, qui a une activité ciblée dans la lutte contre la chute des cheveux et favorise la croissance des cheveux.

De manière surprenante et inattendue, les inventeurs ont découvert que la composition associant la miliacine à une dose plus faible, à des lipides polaires présente une activité très largement supérieure à celle de la miliacine seule, déjà connue. En effet, la miliacine est connue pour accélérer la division cellulaire au niveau du bulbe et activer le métabolisme cellulaire.

Dans la composition selon l'invention, l'association au sein d'une même composition de miliacine et de lipides polaires permet, en présence d'eau comme c'est le cas dans le milieu intestinal, de former des structures capables d'encapsuler la miliacine. Les liposomes, ainsi formés, améliorent la biodisponibilité de la miliacine en favorisant le passage intestinal, comme cela a déjà été décrit précédemment pour d'autres molécules (curcumine, naringénine).

Avantageusement, la miliacine et les lipides polaires sont d'origine végétale.

### Miliacine

La miliacine est un triterpénoïde retrouvé uniquement, à ce jour, dans le millet *Panicum miliaceum.* La miliacine a d'abord été étudiée pour ses propriétés cicatrisantes et son activité sur la prolifération cellulaire. Elle est utilisée depuis quelques temps dans des compléments alimentaires destinés à la beauté et à la santé des cheveux, comme indiqué plus haut.

La miliacine est avantageusement présente dans la composition selon la présente invention dans une proportion massique comprise entre 0,1% et 10 %, notamment dans une proportion supérieure à 0,5 %, étant par exemple inférieure à 2%, notamment à 1 %, par rapport à la masse totale de la composition.

La miliacine est contenue dans une huile de millet, par exemple obtenue par CO₂ supercritique.

Une telle huile est obtenue en broyant des graines de millet *Panicum miliaceum* entières ou décortiquées pour obtenir une farine de millet. Une extraction est alors réalisée, par exemple par CO2 supercritique. L'extrait de millet obtenu est alors séché sous vide et stabilisé par un extrait de romarin afin d'éviter tout risque d'oxydation.

La composition de cet extrait de millet est préférentiellement la suivante : 85 à 99% de triglycérides (dont notamment de l'acide linoléique, de l'acide oléique, et de l'acide palmitique), 0.5 à 2% de stérols (dont du beta-sitostérol, du delta-7-stigmastérol et d'autres stérols), et 0,1 à 2% de miliacine.

### Les lipides polaires

Les lipides polaires constituent une classe à part dans les lipides puisqu'ils possèdent une partie hydrophile qui leur permet de jouer un rôle prépondérant au niveau des interfaces, que ce soit dans les organismes vivants ou dans les systèmes dispersés. Ils sont notamment les constituants principaux des membranes biologiques.

Les sphingolipides, en particulier les céramides et glycosylcéramides, jouent des rôles biologiques de grande importance dans la structure même de l'épiderme par exemple. Dans cette zone, les cornéocytes sont séparés par des espaces intercellulaires, garnis par le ciment intercornéocytaire organisé en multi-feuillets. Ce ciment intercornéocytaire est majoritairement composé de céramides et de glycosylcéramides (35 à 40%) et permet de limiter la perte en eau tout en assurant la cohésion cellulaire de l'épiderme. Des études récentes ont démontré qu'une partie des céramides et des glycosylcéramides ingérées est retrouvée dans la peau. Des études cliniques ont mis en évidence l'activité hydratante des céramides et glycosylcéramides absorbées par voie orale et une amélioration des signes clinques associés à la peau sèche, tels que des rougeurs, des squames ou encore des démangeaisons.

Les céramides sont également présentes dans la cuticule du cheveu où elles assurent la cohésion entre cellules et protègent les cheveux de l'entrée de composés indésirables. Elles ont également un rôle structural et assurent les propriétés mécaniques du cheveu.

Les phospholipides sont des composants clés de la membrane biologique des cellules, ils représentent environ 55% des lipides membranaires. Ils ont la capacité de s'auto-organiser en un double feuillet, leurs têtes hydrophiles pointant vers l'extérieur et leurs chaînes hydrophobes pointant vers l'intérieur de la membrane.

Une autre propriété des lipides polaires, et particulièrement des phospholipides, qui est d'un intérêt particulier dans la présente invention, concerne leurs capacités à servir de vecteur ou support en augmentant la biodisponibilité des composés actifs. En effet, les phospholipides sont reconnus et utilisés pour leur aptitude à former des vésicules. Ces vésicules peuvent permettre d'améliorer la biodisponibilité de certains actifs, en facilitant le passage intestinal.

Selon un mode de réalisation préféré, la proportion en lipides polaires est supérieure à 0,5 % par rapport à la masse totale de la composition.

Avantageusement, les lipides polaires comprennent des sphingolipides, notamment des céramides et/ou des glycosylcéramides. Dans ce cas, la proportion massique de céramides et/ou de glycosylcéramides est avantageusement comprise entre 0,01 % et 5 %, notamment comprise entre 0,1% et 1 % par rapport à la masse totale de la composition, notamment supérieure à 0,1% voire à 0,2% par rapport à la masse totale de la composition.

Les lipides polaires peuvent comprendre également des phospholipides. Dans ce cas, la proportion massique de phospholipides est avantageusement comprise entre 0,01 % et 5 %, notamment entre 0,3% et 1,5 % par rapport à la masse totale de la composition, étant par exemple supérieure à 0,5%, voire à 0,8% par rapport à la masse totale de la composition.

Les lipides polaires, notamment les sphingolipides, tels que les céramides et/ou glycosylcéramides, et/ou les phospholipides, peuvent en particulier être contenus dans un extrait lipidique végétal, dans des extraits de plantes tels que le konjac, ou dans des extraits de céréales et légumineuses riches en lipides polaires, tels que le maïs, le riz, le blé ou le soja, ou bien dans des produits laitiers riches en lipides polaires.

Dans le cas de l'extrait de blé, il s'agit par exemple d'une huile végétale ou d'une poudre lipidique pouvant être obtenue selon le procédé décrit dans le brevet FR 2 785 806 dont le contenu est incorporé à la présente par référence. Le procédé d'extraction décrit dans le brevet FR 2 785 806 utilise en particulier l'éthanol comme solvant.

En particulier, l'extrait de blé peut consister en celui qui est commercialisé sous le nom Lipowheat® par la société HITEX, déposante de la présente.

Le procédé de préparation du Lipowheat® peut comporter l'étape de récolte des grains de blé *Triticum aestivumlvulgare,* suivie d'une lixiviation des grains broyés pour obtenir le gluten d'un côté et l'amidon de l'autre, puis, par extraction éthanolique du gluten de blé, l'obtention de Lipowheat® huile et après une seconde extraction à l'aide d'acétone, l'obtention de Lipowheat® poudre.

### Formation de vésicules en présence de lipides polaires, de miliacine et d'eau

Les observations au microscope optique d'un mélange contenant des lipides polaires apportés par Lipowheat®, de la miliacine sous forme de cristaux, et de l'eau, indiquent clairement la formation de vésicules. Ces structures assez rigides présentent un diamètre compris entre 5 et 25 µm.

Ce système ne s'observe qu'en présence de miliacine. Cette dernière jouant un rôle clé au niveau de l'interface des liposomes qui se rigidifient pour générer de véritables capsules.

Les liposomes, ainsi formés, améliorent la biodisponibilité de la miliacine en favorisant le passage intestinal, comme cela a déjà été décrit précédemment pour d'autres molécules (curcumine, naringénine).

### Composition moyenne de la formule conforme à l'invention

Trois formes de la composition conforme à l'invention sont disponibles :
- une forme huileuse nommée KERANAT
- une forme poudre (1) hydrosoluble nommée KERANAT WS POWDER
- une forme poudre (2) hydrodispersible nommée KERANAT POWDER

### Forme huile : KERANAT

| | Teneur moyenne | Teneur préférentielle |
|---|---|---|
| Acide linoléique | 30 - 70% | 50 - 65% |
| Miliacine | 0,01 - 10% | 0,1 - 2% |
| Phospholipides | 0,01 - 5% | 0,30 - 1,5% |
| Céramides et Glycosylcéramides | 0,01 - 5% | 0,1 - 1% |
| Autres acides gras | 10 - 40% | 15 - 30% |
| Autres lipides | 0,01 - 5% | 0,5 - 3 % |

### Forme poudre 1 : KERANAT WS POWDER

| | Teneur moyenne | Teneur préférentielle |
|---|---|---|
| KERANAT | 10.00 % - 50.00 % | 20.00 % - 30.00 % |
| Maltodextrines | 20.00 % - 60.00 % | 40.00 % - 50.00 % |
| Amidon modifié | 10.00 % - 50.00 % | 30.00 % - 40.00 % |

### Forme poudre 2 : KERANAT POWDER

| | Teneur moyenne | Teneur préférentielle |
|---|---|---|
| KERANAT | 10.00 % - 50.00 % | 20.00 % - 30.00 % |
| Pulpe de baobab | 50.00 % - 90.00 % | 70.00 % - 80.00 % |
| Silice | 0.00 % - 5.00 % | 0.00 % - 2.00 % |

### Autres composants

La composition selon l'invention peut encore être utilisée dans le domaine alimentaire, comme complément alimentaire ou aliment fonctionnel sous toute forme galénique : comprimés, gélules, capsules molles, boissons ou autre.

La composition selon l'invention peut intégrer les formules de compléments alimentaires où elle peut être associée à des composés connus de l'Homme de l'art et ainsi comporter des acides aminés soufrés, des vitamines, notamment des vitamines du groupe B, des minéraux, notamment du cuivre, du zinc ou du sélénium, ou un extrait de plante.

La composition selon l'invention peut également être utilisée sous forme topique sous toute forme galénique : huile, gel aqueux, gel lipidique, émulsion huile dans eau, émulsion eau dans huile, gel moussant et lavant, savon, patch ou lotion ou tout autre concept galénique topique connu par l'Homme de l'art. La composition selon l'invention peut intégrer les formules cosmétiques orale et/ou topiques contenant des lipides, polymères gélifiants et viscosants, des tensioactifs et émulsifiants, des principes actifs hydrosolubles ou liposolubles, ou des extraits d'autres matières premières, habituellement utilisés en cosmétique et connus de l' Homme de l'art.

### Procédé de préparation d'une composition selon l'invention

L'invention a encore pour objet, en combinaison avec ce qui précède, un procédé de préparation de la composition telle que définie plus haut, comprenant les étapes suivantes :
- obtention d'une huile de millet comprenant de la miliacine par extraction,
- obtention d'un composant huileux comprenant des sphingolipides, notamment des glycosylcéramides et/ou des céramides, et/ou des phospholipides par extraction éthanolique, et
- mélange de l'huile de millet et du composant huileux, notamment à pression atmosphérique ou en conditions supercritiques lors de l'extraction du millet.

L'extraction pour l'obtention de l'huile de millet peut par exemple être choisie dans le groupe des techniques suivantes: extraction au fluide supercritique, notamment au CO₂ supercritique ou au propane supercritique, et extraction aux solvants, notamment à l'hexane, à l'éthanol ou à l'isopropanol.

Avantageusement, l'extraction de l'huile de millet est réalisée par la technique d'extraction au CO₂ supercritique. L'extraction selon cette technique a un bon rendement et l'extrait issu de cette technique est de grande qualité.

L'extraction lipidique pour l'obtention du composant huileux comprenant les sphingolipides, notamment les glycosylcéramides et/ou les céramides, et/ou les phospholipides peut être par exemple une extraction aux solvants, notamment à l'éthanol.

Le mélange de l'huile de millet et du composant huileux peut, selon un premier mode de réalisation, être mis en oeuvre à la fin du procédé par mélange après obtention de chacun des composants, à pression atmosphérique notamment.

En variante, selon un deuxième mode de réalisation, préférentiel, le mélange de l'huile de millet et du composant huileux peut être mis en oeuvre par ajout, notamment par injection, du composant huileux dans un circuit d'extraction de l'huile de millet, au cours de l'extraction, notamment au cours de l'extraction par CO₂ supercritique. L'étape de mélange peut être en particulier un dérivé de la technique décrite dans le procédé selon le brevet FR 99 13241.

L'ajout du composant huileux comprenant des lipides polaires dans le circuit d'extraction de l'huile de millet a lieu en milieu supercritique, sans oxygène.

La composition préparée dans un milieu sans oxygène ne subit pas de phénomène d'oxydation et est plus stable. La mise en oeuvre de cette étape du procédé permet ainsi d'obtenir une composition plus homogène et plus stable.

### Utilisation d'une composition selon l'invention

L'invention a encore pour objet, en combinaison avec ce qui précède, l'utilisation d'une composition comprenant au moins de la miliacine dans une proportion supérieure à 0,1% massique par rapport à la masse totale de la composition et lipides polaires, notamment des sphingolipides, tels que des céramides et/ou glycosylcéramides, et/ou des phospholipides, dans une proportion supérieure à 0,1% massique par rapport à la masse totale de la composition, aux fins de lutter contre la chute de cheveux ou de poils d'animaux et/ou pour favoriser leur croissance et/ou leur repousse et/ou améliorer leur beauté (brillance, douceur, force) et /ou améliorer le confort du cuir chevelu.

### 5. Essais

L'invention, ainsi que les avantages qu'elle présente, seront plus facilement compris grâce aux exemples de réalisation de celle-ci exposés ci-après, réalisés *in vitro* et *in vivo.*

### Essai 1 : essai in vitro

### Objectifs de l'essai in vitro

Ce test consiste à étudier la prolifération cellulaire des cellules épithéliales (kératinocytes). En effet, comme indiqué plus haut, la prolifération cellulaire au de la matrice du bulbe du cheveu permet de favoriser la croissance des cheveux.

Ce test a pour objectif de vérifier les éléments décrits dans la littérature scientifique pour la miliacine et d'évaluer l'intérêt d'une association entre la miliacine et les lipides polaires.

### Compositions étudiées :

Composition A : 1,7 µg/ml de miliacine
Composition B : 150 µg/ml de la composition conforme à l'invention, Keranat, comprenant :
   ∘ 1.7 µg/ml de miliacine (1.1 % du mélange)
   ∘ 0.30 µg/ml de glycosylcéramides et céramides (0.21% du mélange)
   ∘ 1.2 µg/ml de phospholipides (0.8% du mélange)

### Méthodologie

Des fragments de scalp humain ont été obtenus en chirurgie plastique à partir de douze donneurs différents de couronne occipitale avant greffe capillaire. Ils ont été déposés dans des inserts, eux-mêmes mis en place sur des puits de culture. Du milieu de culture spécifiquement adapté au maintien en survie (antibiotique, sérum de veau foetal (SVF)) a été ajouté dans le fond des puits, un passage s'effectuant par diffusion lente entre les deux compartiments par l'intermédiaire d'une membrane poreuse d'épaisseur 3 µm.

La composition est ajoutée en dilution à une dose déterminée, dans le milieu de culture afin de favoriser le contact avec les bulbes piliaires.

On a comparé trois séries de scalps au cours de deux tests réalisés successivement :
- un scalp témoin ;
- un scalp sur lequel on a appliqué la composition A comprenant de la miliacine seule ;
- un scalp sur lequel on a appliqué la composition B conforme à l'invention comprenant de la miliacine et des lipides polaires, parmi lesquels des céramides, glycosylcéramides et des phospholipides.

Les cultures ont été stoppées à J4 (quatrième jour) et les fragments de scalps ont été fixés dans le formol pour permettre de réaliser les analyses immuno histochimiques.

### Méthodes d' analyse des résultats

La prolifération épithéliale a été analysée par immunohistochimie à l'aide d'un anti corps anti-Ki67 marqueur des cellules en phase M, S, G1 et G2 du cycle cellulaire (anticorps monoclonal murin, clone MIB1, dilué au 1/300, commercialisé par la société Dako). L'immunodétection a été réalisée à l'aide d'une technique d'immunoperoxydase indirecte en quatre couches, amplifiée (kit CsA, commercialisé par la société Dako) et révélée en rouge par l'AEC (3-amino-9-ethylcarbazole).

Au niveau de chaque bulbe, le nombre de cellules positives est comptabilisé par rapport à un total d'environ 100 cellules. Pour chaque scalp, l'analyse est effectuée sur environ 8 donneurs différents.

### Résultats obtenus

Les résultats obtenus sont les suivants :

| | Zone d'étude | Résultats (% de cellules en prolifération par rapport au contrôle,) |
|---|---|---|
| Composition A Miliacine seule | bulbe | + 92% |
| Composition B Miliacine et lipides polaires | bulbe | +138% |

Le nombre de cellules en prolifération est 92 % plus important dans les bulbes de cheveux en contact avec l'huile de millet seule.

Le nombre de cellules en prolifération est 138 % plus important dans les bulbes de cheveux en contact avec la composition conforme à l'invention.

Le gain de performance, avec la composition conforme à l'invention, associant la miliacine aux lipides polaires, est donc d'environ 50%. De manière surprenante et imprévisible, l'amélioration de la performance de l'efficacité, sur la prolifération cellulaire au niveau du bulbe, de la miliacine par l'addition de lipides polaires, est très significative et sans équivoque.

L'association entre les agents actifs que sont les lipides polaires dont les céramides, glycosylcéramides et les phospholipides, avec la miliacine, dans la composition conforme à l'invention, permet donc, de manière inattendue, une meilleure action sur la prolifération cellulaire au niveau du bulbe, que la miliacine seule.

### Essai 2 : essais in vivo

### Objectifs des essais in vivo

Ces tests consistent à démontrer, dans un premier temps, l'efficacité antichute de la miliacine seule, puis dans un second temps, l'efficacité équivalente d'une dose inférieure de miliacine associée à des lipides polaires dans une association conforme à l'invention.

En effet, la miliacine étant un lipide rare, les inventeurs ont cherché à vérifier si, en diminuant la quantité de miliacine dans la composition tout en ajoutant des lipides polaires, l'effet de la composition serait de même intensité.

### Première étude clinique :

Objectif : évaluer l'efficacité antichute d'une huile de millet extraite par CO₂ supercritique, standardisée en miliacine, sous la forme de capsule molle apportant 350 mg par jour de la formule, soit 3.5 à 4.0 mg/jour de miliacine.

Une première étude clinique a été réalisée sur 60 volontaires, des femmes, de moyenne d'âge 37 ans. Le but de cette étude est de démontrer l'efficacité d'un produit comprenant de la miliacine, comme seul principe actif, sur la chute des cheveux.

### Composition

La composition testée comprend entre 3,5 et 4 mg de miliacine, environ 200 à 250 mg d'acide linoléique et environ 50 à 100 mg d'autres acides gras.

| Molécules | Teneur dans la composition testée |
|---|---|
| Acide linoléique | 200 - 250 mg |
| Miliacine | 3.5 - 4.0 mg |
| Autres acides gras | 50 - 100 mg |
| Dose journalière testée | 350 mg |

### Méthodologie

L'étude a été réalisée en double aveugle, contre placebo et randomisée. Les tests ont eu lieu à un temps t0 et à un temps t12, 12 semaines après t0. Les paramètres observés lors de l'étude sont le nombre de cheveux, le pourcentage de cheveux en phase anagène et en phase télogène, la résistance des cheveux, l'aspect général des cheveux.

La méthode utilisée pour déterminer le pourcentage de cheveux en phase télogène et anagène est le Trichogramme avec le Trichoscan®.

Des premiers résultats significatifs ont été obtenus après trois mois d'utilisation et une consommation quotidienne de 350 mg de produit.

### Résultats

Les résultats indiquent que la composition à base de miliacine diminue d'environ 8 % le pourcentage de cheveux en phase télogène, faisant passer le nombre de cheveux en phase télogène de 21,5% à 19,8% environ.

Le placebo est stable, le nombre de cheveux en phase télogène ne diminuant pas (augmentation très légère de 0,03 %).

La différence entre la composition à base de miliacine et le placebo est significative après trois mois d'utilisation.

### Seconde étude clinique :

Objectif : évaluer l'efficacité antichute d'une association originale d'huile de millet extraite par CO₂ supercritique, standardisée en miliacine et de lipides polaires, sous la forme de capsule molle apportant 300 mg par jour de la formule, soit 3.0 à 3.3 mg/jour de miliacine. Cette étude a pour objectif de démontrer l'efficacité d'une dose plus faible de miliacine lorsque celle-ci est associée à des lipides polaires.

Une seconde étude clinique est réalisée sur 65 femmes avec de l'huile de millet associée à des lipides polaires dont les glycosylcéramides, céramides et phospholipides, correspondant à une composition conforme à l'invention.

### Composition testée

La composition testée comprend entre 3.0 et 3.3 mg de miliacine et entre 0.5 et 0.8 mg de glycosylcéramides et céramides, entre 2 et 4 mg de phospholipides, environ 150 à 200 mg d'acide linoléique et 50 à 100 mg d'autres acides gras.

| Molécules | Teneur dans la composition testée |
|---|---|
| Acide linoléique | 150 - 200 mg |
| Miliacine | 3.0 - 3.3 mg |
| Glycosylcéramides et céramides | 0.5 - 0.8 mg |
| Phospholipides | 2.0 - 4.0 mg |
| Autres acides gras | 50 - 100 mg |
| Dose journalière testée | 300 mg |

### Méthodologie

C'est une étude randomisée en double aveugle contre placebo qui a duré 12 semaines. Les conditions de l'étude ont été en double aveugle, de manière aléatoire, et des analyses ont eu lieu à un temps t0, à un temps t6 et à un temps t12, 12 semaines après t0.

Les paramètres observés de l'étude sont le nombre de cheveux, le pourcentage de cheveux en phase anagène et en phase télogène, l'aspect général des cheveux et du cuir chevelu. La méthode utilisée pour déterminer le pourcentage de cheveux en phase télogène et anagène est le Trichogramme.

### Résultats

Les résultats obtenus sont non seulement, comme espéré, au moins équivalents à ceux de la première étude clinique, mais sont en plus largement supérieurs à ceux de la première étude clinique, avec une amélioration de plus de 500% après trois mois d'utilisation et une consommation quotidienne de 300 mg de composition, alors que la dose ingérée est diminuée d'environ 25% par rapport à la première étude clinique.

En effet, une diminution de 50% environ du pourcentage de cheveux en phase télogène a été observée après trois mois d'utilisation de la composition conforme à l'invention. La composition a ralenti la chute des cheveux de manière significative et très supérieure par rapport à l'étude précédente.

Les résultats obtenus lors de ces deux études cliniques prouvent, sans équivoque et dans des proportions surprenantes, la singularité de l'association de la miliacine avec les lipides polaires. La synergie entre les deux ingrédients est inattendue et l'efficacité démontrée indique une activité antichute très supérieure aux attentes.
La dose de 300 mg de la composition conforme à l'invention, apportant 3 mg de miliacine environ, est 6 fois plus efficace pour diminuer le pourcentage de cheveux en phase télogène, que la miliacine seule à 4 mg dans une huile végétale à une dose de 350 mg.

De façon totalement inattendue, en diminuant la dose journalière de miliacine d'environ 25% et en associant, de manière inédite, la miliacine à des lipides polaires, la performance est multipliée par 6, ce qui est considérable et inespéré.

| | Produit étudié | Dose journalière | RESULTAT |
|---|---|---|---|
| ETUDE 1 | Huile de millet contenant 1 % de miliacine | 350 mg 3.5 à 4.0 mg de miliacine | Diminution de 8 % du pourcentage de cheveux en phase télogène |
| ETUDE 2 | Composition conforme à l'invention contenant de l'huile de millet à 1 % de miliacine et des lipides polaires | 300 mg 3.0 à 3.3 mg de miliacine + lipides polaires | Diminution de 50 % du pourcentage de cheveux en phase télogène |

D'autre part, 91% des femmes qui ont consommé la composition ont vu leur chute de cheveux diminuer. Une diminution de 73% de la présence de pellicules et une diminution de 75% de la sécheresse du cuir chevelu a également été observée.

Cette seconde étude clinique prouve de manière inédite, l'intérêt d'une association, telle que décrite dans ce brevet, entre la miliacine et les lipides polaires pour augmenter les propriétés antichute de la miliacine.

### 5. Exemples

### Formules de compléments alimentaires

### Complément alimentaire A

Il s'agit d'un complément alimentaire sous la forme d'une ou deux capsules molles par jour contenant les ingrédients suivants :

| Ingrédients | Teneur en mg dans la capsule |
|---|---|
| Composition conforme à l'invention KERANAT | 300 mg, apportant 3.0 à 3.3 mg de miliacine environ et 0.5 mg à 0.8 mg de glycosylcéramides et céramides et 2 à 4 mg de phospholipides. |
| Zinc | 10 mg = 100 % des AJR |
| Vitamine B6 | 1.4 mg = 100 % des AJR |
| Vitamine B8 | 50 µg = 100 % des AJR |

### Complément alimentaire B

Il s'agit d'un complément alimentaire sous la forme de deux capsules molles par jour contenant les ingrédients suivants :

| Ingrédients | Teneur en mg dans deux capsules |
|---|---|
| Composition conforme à l'invention KERANAT | 300 mg, apportant 3.0 à 3.3 mg de miliacine environ et 0.5 mg à 0.8 mg de glycosylcéramides et céramides et 2 à 4 mg de phospholipides. |
| Huile de pépins de courge | 500 mg |
| Cystine | 20 mg |
| Vitamine B5 | 10 mg = 100 % des AJR |

### Complément alimentaire C

Il s'agit d'un complément alimentaire sous la forme d'une ou deux capsules molles par jour contenant les ingrédients suivants :

| Ingrédients | Teneur en mg dans la capsule |
|---|---|
| Composition conforme à l'invention KERANAT | 300 mg, apportant 3.0 à 3.3 mg de miliacine environ et 0.5 mg à 0.8 mg de glycosylcéramides et céramides et 2 à 4 mg de phospholipides. |
| Kératine | 100 mg |
| Zinc | 10 mg = 100 % des AJR |
| Vitamine B6 | 1.4 mg = 100 % des AJR |

### Complément alimentaire D

Il s'agit d'un complément alimentaire sous la forme d'un stick par jour contenant les ingrédients suivants :

| Ingrédients | Teneur en mg dans le sachet ou le stick |
|---|---|
| KERANAT WS POWDER | 1.5 g apportant 3.0 à 3.3 mg de miliacine environ et 0.5 mg à 0.8 mg de glycosylcéramides et céramides et 2 à 4 mg de phospholipides. |
| Kératine | 500 mg |
| Cystine | 20 mg |
| Zinc | 10 mg = 100 % des AJR |
| Vitamine B6 | 1.4 mg = 100 % des AJR |
| Vitamine B5 | 10 mg = 100 % des AJR |
| Vitamine B8 | 50 µg = 100 % des AJR |

### Complément alimentaire E

Il s'agit d'un complément alimentaire sous la forme d'un stick ou d'un sachet par jour contenant les ingrédients comme suit :

| Ingrédients | Teneur en mg dans le sachet ou le stick |
|---|---|
| KERANAT POWDER | 1.5 g apportant 3.0 à 3.3 mg de miliacine environ et 0.5 mg à 0.8 mg de glycosylcéramides et céramides et 2 à 4 mg de phospholipides. |
| Extrait de thé vert | 300 mg |
| Taurine | 150 mg |
| Cystine | 150 mg |
| Zinc | 10 mg = 100 % des AJR |
| Vitamine B6 | 1.4 mg = 100 % des AJR |
| Vitamine B5 | 10 mg = 100 % des AJR |
| Vitamine B8 | 50 µg = 100 % des AJR |

### Formules de cosmétiques

Chez l'Homme la peau recouvre l'ensemble du corps pour une superficie totale de 2 m2 environ, celle-ci pèse environ 5 kg. Le cuir chevelu recouvre environ 600 cm2 soit environ 1/33^{e} de la peau de notre corps, pour une masse d'environ 0,15 kg. Le poids moyen d'une femme étant d'environ 63 kg, la masse du cuir chevelu représente donc environ 1/420^{e} de la masse du corps.

Il a été démontré que, 300 mg/jour de la composition selon l'invention, avait sur une femme de poids moyen, un effet sur la chute des cheveux, sur leur croissance et/ou leur repousse et/ou sur l'amélioration de la beauté et /ou sur l'amélioration du confort du cuir chevelu.

La masse du cuir chevelu est 420 fois inférieure à celle de la masse corporelle totale. Si on utilise ce même facteur pour déterminer la dose équivalente à la dose efficace par voie orale, soit 300 mg, on arrive à 0.7 mg de la composition conforme à l'invention, par jour et par dose d'application (si une seule application par jour).

L'application d'une lotion par exemple en spray devra donc pouvoir dispenser cette quantité dans un 1 ml de produit, cette quantité correspondant à une application moyenne. Or, 1 ml de solution aqueuse pèse environ 1 g ; 0,7 mg correspond ainsi à 0,07% M/M d'actif dans le produit fini.

La perte occasionnée par le passage transdermique et les attaques enzymatiques à la surface de la peau est généralement anticipée par l'Homme de l'art. En augmentant la concentration d'un facteur 10, la concentration en produit conforme à l'invention est ramenée à 0,7% M/M d'une formule cosmétique.

### Formule 1 - shampoing à usage quotidien

| **Fournisseur** | **Ingrédient** | **Matière active** | **%** |
|---|---|---|---|
| MASSO | Sulfetal C90E | Sodium Cocosulfate | 8,50 |
| BASF | DEHYTON MC | Sodium Cocoamphoacetate | 8,50 |
| UNIPEX | AMISOFT HS11P | Sodium Stearoyl Glutamate | 1,00 |
| BRENNTAG | MANNITOL | Mannitol | 3,00 |
| EPHYLA SAS | Frametime CX | Bentonite & Xanthan gum & Citric acid | 5,50 |
| EPHYLA SAS | CHITOVEG | Chitosan | 0,25 |
| BALLU CHIMIE | AEC8G | Capryloyl Glycine | 0,40 |
| BRENNTAG | Salicylic acid | Salicylic acid | 0,20 |
| MLW | Benzyl Alcohol | Benzyl alcohol | 0,60 |
| BRENNTAG | Sodium Benzoate | Sodium Benzoate | 0,50 |
| HITEX | Composition conforme à l'invention KERANAT | Miliacine 1% ; Phospholipides 1% ; Glycosylceramides/ceramides 0,2% | 0,70 |
| EPHYLA SAS | Huile de dattier du désert | Balanites roxburghii seed oil | 2,00 |
| | Parfum | Parfum | 0,40 |
| | EAU | Aqua | QSP |

Dans toute la description y compris les revendications, les plages de valeurs doivent être comprises comme incluant les bornes, sauf si le contraire est spécifié.

Dans toute la description y compris les revendications, les expressions « comprenant un » et « comportant un » doivent être comprises comme étant synonymes respectivement des expressions « comprenant au moins un » et « comportant au moins un » sauf si le contraire est spécifié.

## Revendications

1. Composition cosmétique non-thérapeutique ou alimentaire pour lutter contre la chute de cheveux ou de poils d'animaux et/ou pour favoriser leur croissance et/ou leur repousse et/ou améliorer leur beauté (brillance, douceur, force) et /ou améliorer le confort du cuir chevelu, comprenant au moins de la miliacine dans une proportion supérieure à 0,1% massique par rapport à la masse totale de la composition et des lipides polaires dans une proportion supérieure à 0,1% massique par rapport à la masse totale de la composition dont des sphingolipides, notamment des glycosylcéramides et/ou des céramides, et/ou des phospholipides dans une proportion supérieure à 0,1% massique par rapport à la masse totale de la composition.

2. Composition selon la revendication 1, dans laquelle la proportion massique en sphingolipides, notamment en céramides et glycosylcéramides, est supérieure à 0,1 %, voire à 0,2%, par rapport à la masse totale de la composition.

3. Composition selon l'une des revendications 1 et 2, dans laquelle la proportion massique en phospholipides est supérieure à 0,5 %, notamment à 0,8% par rapport à la masse totale de la composition.

4. Composition selon l'une quelconque des revendications 1 à 3, dans laquelle les céramides et/ou glycosylcéramides sont contenus dans un extrait de blé.

5. Procédé de préparation de la composition selon l'une quelconque des revendications 1 à 4, comprenant les étapes suivantes :
• obtention d'une huile de millet comprenant de la miliacine par extraction,
• obtention d'un composant huileux comprenant des sphingolipides, notamment des glycosylcéramides et/ou céramides, et/ou des phospholipides par extraction éthanolique, et
• mélange de l'huile de millet et du composant huileux.

6. Procédé de préparation selon la revendication 5, dans lequel l'extraction pour l'obtention de l'huile de millet est choisie dans le groupe des techniques suivantes: extraction au fluide supercritique, notamment au CO2 supercritique ou au propane supercritique, et extraction aux solvants, notamment à l'hexane, à l'éthanol ou à l'isopropanol.

7. Procédé de préparation selon l'une des revendications 5 et 6, dans lequel l'extraction lipidique pour l'obtention du composant huileux comprenant les sphingolipides, notamment les glycosylcéramides et/ou céramides, et/ou les phospholipides est une extraction aux solvants, notamment à l'éthanol.

8. Procédé de préparation selon l'une quelconque des revendications 5 à 7, dans lequel le mélange de l'huile de millet et du composant huileux est mis en oeuvre par ajout, notamment par injection, du composant huileux dans un circuit d'extraction de l'huile de millet, au cours de ladite extraction, notamment au cours de l'extraction par CO2 supercritique.

9. Procédé de préparation selon la revendication précédente, dans lequel l'ajout du composant huileux dans le circuit d'extraction de l'huile de millet a lieu en milieu supercritique, sans 02.

10. Utilisation non-thérapeutique d'une composition comprenant au moins de la miliacine dans une proportion supérieure à 0,1% massique par rapport à la masse totale de la composition et des lipides polaires, notamment des sphingolipides, tels que des céramides et/ou glycosylcéramides, et/ou des phospholipides, dans une proportion supérieure à 0,1% massique par rapport à la masse totale de la composition, aux fins de lutter contre la chute de cheveux ou de poils d'animaux et/ou pour favoriser leur croissance et/ou leur repousse et/ou améliorer leur beauté (brillance, douceur, force) et /ou améliorer le confort du cuir chevelu.

## Patentansprüche

1. Kosmetische nicht therapeutische oder Lebensmittelzusammensetzung zur Bekämpfung von Haar- oder Tierhaarausfall und/oder zur Förderung ihres Wachstums und/oder ihres Nachwachsen und/oder um ihre Schönheit zu verbessern (Glanz, Weichheit, Stärke) und/oder um den Komfort der Kopfhaut zu verbessern, wobei die Zusammensetzung zumindest Miliacin in einem Anteil von mehr als 0,1 Massen-% bezogen auf die Gesamtmasse der Zusammensetzung und polare Lipide in einem Anteil von mehr als 0,1 Massen-% bezogen auf die Gesamtmasse der Zusammensetzung umfasst, einschließlich Sphingolipide, insbesondere Glycosylceramide und/oder Ceramide und/oder Phospholipide in einem Anteil von mehr als 0,1 Massen-%, bezogen auf die Gesamtmasse der Zusammensetzung.

2. Zusammensetzung nach Anspruch 1, wobei der Massenanteil von Sphingolipiden, insbsondere von Ceramiden und Glycosylceramiden größer als 0,1% oder sogar 0,2%, bezogen auf die Gesamtmasse der Zusammensetzung ist.

3. Zusammensetzung nach einem der Ansprüche 1 und 2, wobei der Massenanteil von Phospholipiden größer als 0,5%, insbesondere 0,8%, bezogen auf die Gesamtmasse der Zusammensetzung ist.

4. Zusammensetzung nach einem der Ansprüche 1 bis 3, wobei die Ceramide und/oder Glycosylceramide in einem Weizenextrakt enthalten sind.

5. Verfahren zur Herstellung der Zusammensetzung nach einem der Ansprüche 1 bis 4 die folgenden Schritte umfassend:
• Erhalten eines Miliacin umfassenden Hirseöls durch Extraktion
• Erhalten einer öligen, Sphingolipide, insbesondere Glycosylceramide und/oder Ceramide und/oder Phospholipide umfassenden Komponente durch Ethanolextraktion, und
• Mischen von Hirseöl und der öligen Komponente.

6. Herstellungsverfahren nach Anspruch 5, wobei die Extraktion zur Gewinnung von Hirseöl aus der Gruppe der folgenden Techniken ausgewählt ist: Extraktion mit überkritischem Fluid, insbesondere überkritischem CO2 oder überkritischem Propan, und Lösungsmittelextraktion, insbesondere mit Hexan, Ethanol oder Isopropanol.

7. Herstellungsverfahren nach einem der Ansprüche 5 und 6, wobei die Lipidextraktion zum Erhalt der öligen, Sphingolipide, insbesondere Glycosylceramide und/oder Ceramide und/oder Phospholipide umfassenden Komponente eine Lösungsmittelextraktion ist, insbesondere mit Ethanol.

8. Herstellungsverfahren gemäß einem derAnsprüche 5 bis 7, wobei die Mischung aus Hirseöl und der öligen Komponente durch Hinzufügen der öligen Komponente insbesondere durch Injektion der öligen Komponente in einem Extraktionskreislauf des Hirseöls während der Extraktion, insbesondere während der Extraktion mit überkritischem CO2 durchgeführt wird.

9. Herstellungsverfahren nach dem vorhergehenden Anspruch, wobei das Hinzufügen der öligen Komponente in den Extraktionskreislauf des Hirseöls in einem überkritischen Medium ohne 02 stattfindet.

10. Nicht therapeutische Verwendung einer Zusammensetzung, die mindestens Miliacin in einem Anteil von mehr als 0,1 Massen-% bezogen auf die Gesamtmasse der Zusammensetzung und polare Lipide, insbesondere Sphingolipide, wie Ceramide und/oder Glycosylceramide und/oder Phospholipide in einem Anteil von mehr als 0,1 Massen-%, bezogen auf die Gesamtmasse derZusammensetzung umfasst, zur Bekämpfung von Haar- oder Tierhaarausfall und/oder zur Förderung ihres Wachstums und/oder ihres Nachwachsens und/oder zur Verbesserung ihrer Schönheit (Glanz, Weichheit, Stärke) und/oder um den Komfort der Kopfhaut zu verbessern.

## Claims

1. A non-therapeutic cosmetic or dietary composition to reduce hair or animal hair loss and/or to promote their growth and/or regrowth and/or to improve their beauty (brightness, softness, strength) and/or to improve the comfort of the scalp, comprising at least miliacin in a proportion greater than 0.1% by weight based on the total weight of the composition and polar lipids in a proportion greater than 0.1% by weight based on the total weight of the composition including sphingolipids, notably glycosylceramides and/or ceramides, and/or phospholipids in a proportion greater than 0.1% by weight based on the total weight of the composition.

2. The composition according to claim 1, wherein the proportion by weight in sphingolipids, notably in ceramides and glycosylceramides, is greater than 0.1%, or even greater than 0.2%, based on the total weight of the composition.

3. The composition according to either one of claims 1 or 2, wherein the proportion by weight in phospholipids is greater than 0.5%, notably greater than 0.8%, based on the total weight of the composition.

4. The composition according to any one of claims 1 to 3, wherein ceramides and/or glycosylceramides are contained in an extract of wheat.

5. A method for preparing the composition according to any one of claims 1 to 4, comprising the following steps:
- obtaining a millet oil comprising miliacin by extraction;
- obtaining an oily component comprising sphingolipids, notably glycosylceramides and/or ceramides, and/or phospholipids by extraction with ethanol, and
- mixing the millet oil and the oily component.

6. The method according to claim 5, wherein the extraction for obtaining the millet oil is selected from the group of the following techniques: extraction with supercritical fluid, notably with supercritical CO2 or supercritical propane, and extraction with solvents, notably with hexane, with ethanol or with isopropanol.

7. The method according to either one of claims 5 or 6, wherein lipid extraction for obtaining the oily component comprising sphingolipids, notably glycosylceramides and/or ceramides, and/or phospholipids is an extraction with solvents, notably with ethanol.

8. The method according to any one of claims 5 to 7, wherein the mixture of the millet oil and the oily component is carried out by adding, notably by injection, the oily component in an extraction circuit of the millet oil, during said extraction, notably during extraction with supercritical CO2.

9. The method according to the previous claim, wherein the addition of the oily component in the extraction circuit of the millet oil takes place in an oxygen-free supercritical medium.

10. Non-therapeutic use of a composition comprising at least miliacine in a proportion greater than 0.1 % by weight based on the total weight of the composition and polar lipids, notably sphingolipids, such as ceramides and/or glycosylceramides, and/or phospholipids, in a proportion greater than 0.1% by weight based on the total weight of the composition, for the purpose of reducing hair or animal hair loss and/or to promote their growth and/or regrowth and/or to improve their beauty (brightness, softness, strength) and/or to improve the comfort of the scalp.
